# EUROPÄISCHE PATENTSCHRIFT

(11) **EP 3 429 639 B1**
(45) Veröffentlichungstag und Bekanntmachung des Hinweises auf die Patenterteilung: **29.04.2026**
(21) Anmeldenummer: 17705028.3
(22) Anmeldetag: 25.01.2017
(51) Int. Cl.: A61L 2/10, B67B 3/00, B67B 3/06

(54) **ANORDNUNG ZUM DESINFIZIEREN VON DOSENDECKELN ZUM VERSCHLIESSEN VON DOSEN**
ARRANGEMENT FOR DISINFECTING CAN LIDS FOR CLOSING CANS
ENSEMBLE DESTINÉ À DÉSINFECTER DES COUVERCLES DE BOÎTES POUR FERMER DES BOÎTES

(30) Priorität: 16.03.2016 DE 102016104859
(43) Veröffentlichungstag der Anmeldung: 23.01.2019
(73) Patentinhaber: Claranor SA, 84916 Avignon Cedex 9 (FR); KHS GmbH, 44143 Dortmund (DE)
(72) Erfinder: CLÜSSERATH, Ludwig, 55543 Bad Kreuznach (DE); RIEDEL, Christophe, 84000 Avignon (FR)
(74) Vertreter: BSB Patentanwälte Schütte & Engelen Partnerschaft mbB
(86) Internationale Anmeldenummer: PCT/EP2017/051459
(87) Internationale Veröffentlichungsnummer: WO 2017/157550

(56) Entgegenhaltungen:
- EP-A1- 1 518 565
- EP-A1- 2 650 022
- WO-A1-2005/005260
- WO-A1-2012/069101
- GB-A- 1 093 751
- JP-A- H04 242 525
- JP-A- S5 991 959

## Beschreibung

Die vorliegende Erfindung betrifft eine Anordnung zum Desinfizieren von Dosendeckeln und zum Verschließen von Dosen. Für diese Aufgabe werden bislang getrennte Vorrichtungen vorgesehen, die an unterschiedlichen Positionen einer Füllanlage für die Behälter angeordnet sind. Ein Problem bei diesen bekannten Anordnungen besteht darin, dass die sterilisierten Dosendeckel bis zur Zufuhr zum Dosenverschließer steril gehalten werden müssen. Die Strecke muss daher umkleidet sein und ggf. muss eine entsprechende Steril-Gaszufuhr im Transportbereich vorgesehen sein, die verhindert, dass Keime über die Umgebungsluft auf die Dosendeckel gelangen.

Mit der GB 1 093 751 A ist ein Verfahren und eine Vorrichtung zum Sterilisieren mittels UV-Licht bekannt geworden. Dabei werden Flaschen liegend antransportiert und in einer nach außen geschlossenen langgestreckten Kammer aufgerichtet. Die noch leeren Flaschen werden mit einem Schüttgut befüllt und entlang der Transportstrecke weiter transportiert. Flaschenverschlüsse werden aufeinander gestapelt bevorratet und seitlich von UV-Lampen zur Sterilisation beleuchtet. Schließlich werden die Flaschenverschlüsse aufgesetzt.

Die JP H04 242525 A zeigt ein Verfahren und eine Vorrichtung zur Sterilisation von Behälterdeckeln, die zwischen zwei UV-Lampen beleuchtet werden. Die Vorrichtung umfasst eine langgestreckte lineare Transportstrecke mit einer Transportkette, um die Behälterdeckel an den UV-Lampen vorbei zu transportieren.

Die WO 2005/005260 A1 betrifft ein Verfahren und eine Vorrichtung zum Aufbringen von Werbeträgern auf Verschlüsse von Flaschen wie Mineralwasserflaschen, Fruchtsaftflaschen, Bierflaschen oder Limonadeflaschen, wobei die Verschlüsse mit Etiketten versehen und anschließend entkeimt oder desinfiziert werden. Die fertig behandelten Verschlüsse werden in Vorratsbehälter eingefüllt und dort keimfrei bevorratet.

Die EP 2 650 022 A1 offenbart eine Vorrichtung und ein Verfahren zum strahlungsbasierten Sterilisieren von Behältnisverschlüssen, wobei dreidimensional geformte Behältnisverschlüsse wie Flaschendeckel sterilisiert werden. Dazu muss sichergestellt werde, dass auch das Innere eines Flaschendeckels ausreichend sterilisiert wird, weshalb die Flaschendeckel schräg aufgestellt und schräg dazu angeleuchtet werden.

Die WO 2012/069101 A1 zeigt eine Vorrichtung zum Sterilisieren von Verschlüssen von Behältern. Dabei werden mehrere Verschlüsse vertikal übereinander zu einem Stapel angeordnet. Konzentrisch ist eine Vielzahl säulenförmiger Stapel auf dem Umfang verteilt angeordnet. Radial weiter außen und radial weiter innen sind jeweils eine Vielzahl von UV-Lampen angeordnet, die die Verschlüsse kontinuierlich bestrahlen und dadurch sterilisieren. Die Verschlüsse sind senkrecht ausgerichtet und übereinander gestapelt, um sich nicht gegenseitig abzuschatten. Ein Verschließen der Behältnisse ist nicht direkt vorgesehen.

Mit der JP S59 91959 A ist eine Vorrichtung bekannt geworden, bei der kreisförmige Platten mit UV-Strahlung sterilisiert werden. Die kreisförmigen Platten werden zentral dicht übereinander plaziert. Von der Seite erfolgt eine Bestrahlung mit UV-Licht.

Der Stand der Technik offenbart verschiedene Vorrichtungen und Verfahren zum Desinfizieren von Behälterverschlüssen und Verschlißen von Behältern. Nachteilig ist, dass die Erhaltung der Keimfreiheit aufwändig ist und dass die Vorrichtungen viel Raum benötigen.

Es ist daher Aufgabe der Erfindung, eine Anordnung zu schaffen, bei welcher die Keimfreiheit der Dosendeckel vor dem Verschließen besser gewahrt ist.

Diese Aufgabe wird erfindungsgemäß durch eine Anordnung gemäß Anspruch 1 gelöst. Vorteilhafte Weiterbildungen der Erfindung sind Gegenstand der Unteransprüche.

Erfindungsgemäß enthält die Anordnung eine Zufuhrvorrichtung, z.B. einen Transportstern, in welcher Aufnahmen, z.B. Transportausnehmungen, für vereinzelte Dosendeckel angeordnet sind. Die Zufuhrvorrichtung ist im Zufuhrbereich eines Dosenverschließers angeordnet. Erfindungsgemäß enthält die Anordnung wenigstens eine Sterilisationseinrichtung für die in der Zufuhrvorrichtung aufgenommenen vereinzelten Dosendeckel, welche Sterilisationseinrichtung im Transportbereich der Zufuhrvorrichtung angeordnet ist. Der Vorteil der Erfindung liegt darin, dass die Sterilisationseinrichtung direkt in Verbindung mit der Zufuhrvorrichtung für den Dosenverschließer angeordnet ist. Dadurch laufen die sterilisierten Dosendeckel nur noch über eine minimale Förderstrecke, bevor sie, insbesondere von der Zufuhrvorrichtung, dem Dosenverschließer zugeführt werden. Erfindungsgemäß umfasst die Zufuhrvorrichtung einen Transportstern und die Sterilisationseinrichtung weist zumindest eine Puls-Licht-Vorrichtung auf und es ist jeweils eine Puls-Licht -Vorrichtung über und/oder unter der Zufuhrvorrichtung ausgebildet.

Vorzugsweise enthält die Sterilisationseinrichtung jeweils einen Sterilisierer, z.B. UV-Strahler für die Deckelober- und Unterseite. Auf diese Weise wird der Dosendeckel beidseitig sterilisiert, wobei es als Minimalanforderung ausreichend wäre, dass der Dosendeckel nur auf seiner der Dose zugewandten Unterseite sterilisiert wird, weil dies die Hygiene des in der Dose abgefüllten Produktes, beispielsweise eines Getränks beeinflusst. Die Sterilisationseinrichtung kann selbstverständlich auch mit Gas, mit Chemikalien und/oder Hitze arbeiten. Die Verwendung eines UV-Strahlers beeinflusst die technische Gesamtkonzeption der erfindungsgemäßen Anordnung am wenigsten.

Vorzugsweise ist die Zufuhrvorrichtung als Transportstern ausgebildet, der insbesondere durch einen Motor in seiner Rotationsbewegung gesteuert ist. Ein Transportstern ist technisch einfach zu realisieren und bewirkt eine sichere Aufnahme und Zufuhr der Dosendeckel zum Dosenverschließer, wobei der Transportstern an seinem Umfang auch genug Raum für die Anordnung der wenigstens einen Sterilisationseinrichtung aufweist.

Vorzugsweise ist der Dosenverschließer in dem Transportbereich des Transportsterns angeordnet, so dass die sterilisierten Dosendeckel direkt von dem Zufuhrstern auf die Verschlussposition des Dosenverschließers überführt werden. Die Förderstrecke des sterilisierten Dosendeckels ist somit auf einen bestimmten Sektor des Transportsterns reduziert. Dies ermöglicht eine hohe Sterilität des Dosendeckels beim Verschließen.

Vorzugsweise hat der Zufuhrstern auf seinem Umfangsabschnitt Transportausnehmungen für die vereinzelten Dosendeckel, welche die Aufnahmen für die Dosendeckel bilden. Eine derartige Anordnung ist leicht zu realisieren und bewirkt eine sicherere Halterung der Dosendeckel während des Sterilisierens und evtl. beim Aufbringen auf die Dose im Dosenverschließer. Zudem können Dosendeckel einfach von einer Stapeleinrichtung auf den Transportstern überführt bzw. von dem Transportstern auf eine Stapeleinrichtung überführt werden.

Vorzugsweise enthält die Sterilisationseinrichtung zumindest eine Pulslichtvorrichtung, die berührungslos über starke Lichtimpulse, vorzugsweise im UV-Bereich die Deckel an den zu sterilisierenden Stellen bestrahlt und damit für eine Keimfreiheit sorgt. Der Vorteil einer derartigen Sterilisationseinrichtung ist auch, dass sie den Verlauf und den Transport der vereinzelten Dosendeckel in den Transportstern in keiner Weise beeinträchtigen.

Vorzugsweise ist im Eingang der Zufuhrvorrichtung eine erste Stapeleinrichtung für Dosendeckel angeordnet, so dass die Dosendeckel von der ersten Stapeleinrichtung vereinzelt auf den Transportstern überführt werden können.

In einer vorteilhaften Weiterbildung der Erfindung ist am Ausgang der Zufuhrvorrichtung, z.B. des Transportsterns, eine zweite Stapeleinrichtung angeordnet, die in der Zufuhr des Dosenverschließers liegt. Vorzugsweise bildet dann die zweite Stapeleinrichtung die Zufuhr zum Dosenverschließer. Auch dies erlaubt eine sehr unmittelbare Zufuhr der sterilisierten Dosendeckel zum Dosenverschließer, wobei der Vorteil dieser Anordnung darin liegt, dass durch die zwischen der Zufuhrvorrichtung und dem Dosenverschließer angeordnete zweite Stapeleinrichtung ein Puffer gebildet wird, so dass kurzzeitige Schwankungen in der Sterilisations- und/oder Arbeitsgeschwindigkeit des Dosenverschließers ausgeglichen werden können, natürlich begrenzt durch die Stapelkapazität der zweiten Stapeleinrichtung.

Vorzugsweise ist der Dosenverschließer unmittelbar in oder am Ausgang der Zufuhrvorrichtung angeordnet, so dass die Dosendeckel keine langen Förderwege mehr durchlaufen müssen, nachdem sie von der Zufuhrvorrichtung abgegeben worden sind.

In einer vorteilhaften Weiterbildung der Erfindung ist der Dosenverschließer an der Zufuhrvorrichtung angeordnet und die Zufuhrvorrichtung führt dem Dosenverschließer die sterilisierten Dosendeckel vereinzelt zu. In diesem Fall agiert die Zufuhrvorrichtung bereits als Vereinzelungsvorrichtung für die Zufuhr zum Dosenverschließer. Die Zufuhrvorrichtung erfüllt damit gleichzeitig zwei Aufgaben, nämlich die Vereinzelung und Sterilisierung der Dosendeckel als auch die Zuführung in den Dosenverschließer.

Vorzugsweise sind die Zufuhrvorrichtung und die Sterilisationseinrichtung in einem geschlossenen Gehäuse angeordnet welches eine Zu- und Abfuhr für ein steriles Gas aufweist und natürlich auch eine Zufuhröffnung und Abfuhröffnung für die Dosendeckel. Auf diese Weise kann der gesamte Prozess in einem keimfreien Raum durchgeführt werden, was die Keimfreiheit der im Dosenverschließer aufzubringenden Dosendeckel erhöht.

In einer vorteilhaften Weiterbildung der Erfindung ist die erste Stapeleinrichtung in der Zufuhröffnung angeordnet, und/oder die zweite Stapeleinrichtung ist in der Abfuhröffnung angeordnet. Dies schafft viel Raum im Gehäuse und erlaubt eine einfach zu- und Abfuhr der Dosendeckel.

Vorzugsweise werden die Öffnungen, welche für die Zuführung oder Abführung der Dosendeckel vorgesehen sind, auch für die Gaszufuhr- und -abfuhr verwendet. Auf diese Weise kann die Anzahl der Öffnungen in dem ansonsten geschlossenen Gehäuse minimiert werden, was die Erhaltung der Keimfreiheit verbessert. Dabei ist es nicht zwingend, dass die Zufuhröffnung für die Dosendeckel auch als Zufuhröffnung für das Gas verwendet wird.

Folgende Ausdrücke werden synonym verwendet: Gaszufuhr - Gaszufuhrkanal; Dosendeckel - Deckel; Ausgangsrohr - Gasabfuhr - Dosendeckelabfuhröffnung; Sterilisationseinrichtung - Sterilisierer - UV-Strahler
Es ist für den Fachmann offensichtlich, dass die o.g. Ausführungsformen in beliebiger Weise miteinander kombiniert werden können.

Die Erfindung wird nun beispielsweise anhand eines Ausführungsbeispiels im Zusammenhang mit der schematischen Zeichnung beschrieben.

In dieser zeigen:
- Fig. 1: eine perspektivische Ansicht einer erfindungsgemäßen Anordnung mit einer zwischen der Zufuhrvorrichtung und dem Dosenverschließer angeordneten Stapeleinrichtung;
- Fig. 2: einen vertikalen Schnitt durch die Anordnung gemäß Fig. 1 und
- Fig. 3: einen Schnitt III-III aus Fig. 2.

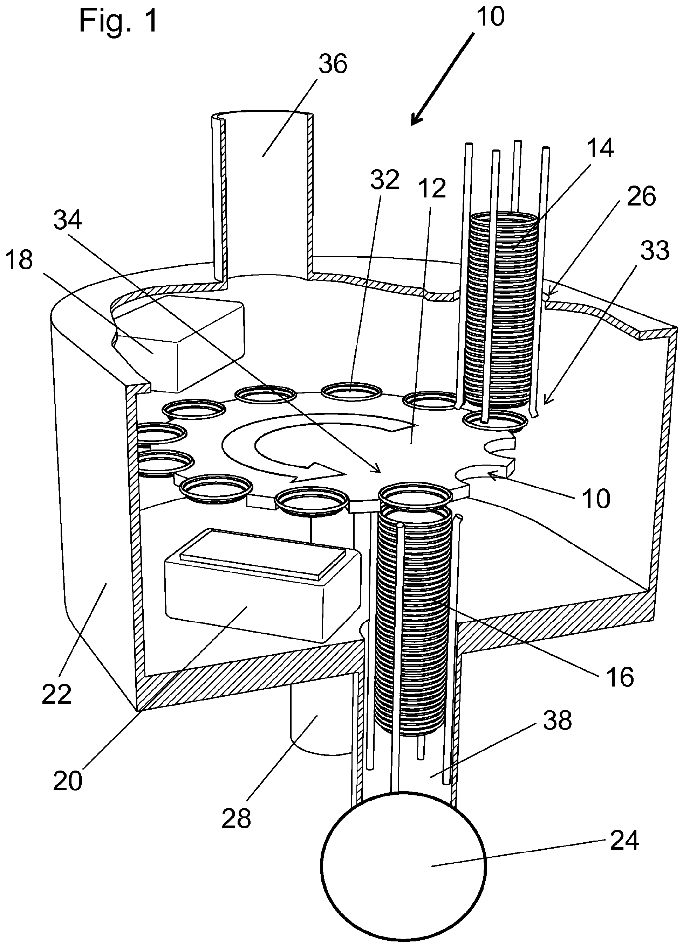

Die erfindungsgemäße Anordnung 10 umfasst gemäß den Figuren 1 bis 3 eine Zufuhrvorrichtung 12 in Form eines Transportsterns, der durch einen Motor 28 angetrieben ist. Der Transportstern 12 ist zusammen mit einer ersten Stapeleinrichtung 14, einer zweiten Stapeleinrichtung 16, einem oberen UV-Strahler 18 und einem unteren UV-Strahler 20 in einem weitgehend geschlossenen Gehäuse 22 angeordnet. Die erste Stapeleinrichtung 14 ist in einer Zufuhröffnung 26 des Gehäuses 22 angeordnet. Die zweite Stapeleinrichtung 16 ist in einem Ausgangsrohr 38 angeordnet, welches aus dem Gehäuse 22 heraus ragt. Das Ausgangsrohr 38 bildet auch eine Gasabfuhr für ein in dem Gehäuse 22 verwendetes Prozessgas, z.B. ein keimfreies oder sterilisierendes Spülgas. Am Ausgang der zweiten Stapeleinrichtung 16 ist ein Dosenverschließer 24 angeordnet.

An seinem Umfang hat der Transportstern 12 Transportausnehmungen 30, in denen vereinzelte Dosendeckel 32 aufgenommen sind. Die Rotationsrichtung des Transportsterns 12 ist durch einen Pfeil dargestellt. Die Gaszu- und -abfuhr 36, 38 kann optional vorgesehen sein. Das Gehäuse ist vorzugsweise bis auf die Zufuhröffnung 26 für die Dosendeckel, den Gaszufuhrkanal 36, und das Ausgangsrohr 38, welches sowohl die Dosenabfuhröffnung als auch die Gasabfuhr bildet, komplett geschlossen.

Die erfindungsgemäße Anordnung arbeitet wie folgt:
Die Dosendeckel 32 werden am Zufuhrpunkt 33 dem Transportstern 12 über die erste Stapeleinrichtung 14 zugeführt. Am Ausgang der ersten Stapeleinrichtung 14 ist eine nicht dargestellte Vereinzelungseinrichtung angeordnet, welche dem Transportstern, d.h. den Transportausnehmungen 30 des Transportsterns 12 die Dosendeckel 32 vereinzelt zuführt. Die Deckel werden dann in den Transportausnehmungen 30 des Transportsterns 12 transportiert, und zwar unter dem oberen UV-Strahler 18 hindurch und über dem unteren UV-Strahler 20 entlang, so dass die Dosendeckel 32 sowohl auf ihrer Oberseite als auch auf ihrer Unterseite sterilisiert werden. Schließlich werden die Dosendeckel am Abfuhrpunkt 34 der zweiten Stapeleinrichtung 16 zugeführt, welche die Zufuhrvorrichtung für den darunter angeordneten Dosenverschließer 24 bildet. Zudem wird dem Gehäuse 22 über den Gaszufuhrkanal 36 ein insbesondere sterilisierendes Gas zugeführt, das an dem Ausgangsrohr 38 abgeführt wird, in welchem die zweite Stapeleinrichtung 16 angeordnet ist. Auf diese Weise werden die Dosendeckel 32 während des gesamten Vereinzelungs-, Sterilisations- und Zufuhrprozesses mit einem keimfreien bzw. sterilisierenden Gas umspült, so dass die durch die UV-Strahler 18, 20 verursachte Sterilisation bzw. Keimfreiheit bis zum Verschließen einer Dose mit einem sterilisierten Dosendeckel 32 im Dosenverschließer 24 erhalten bleibt.

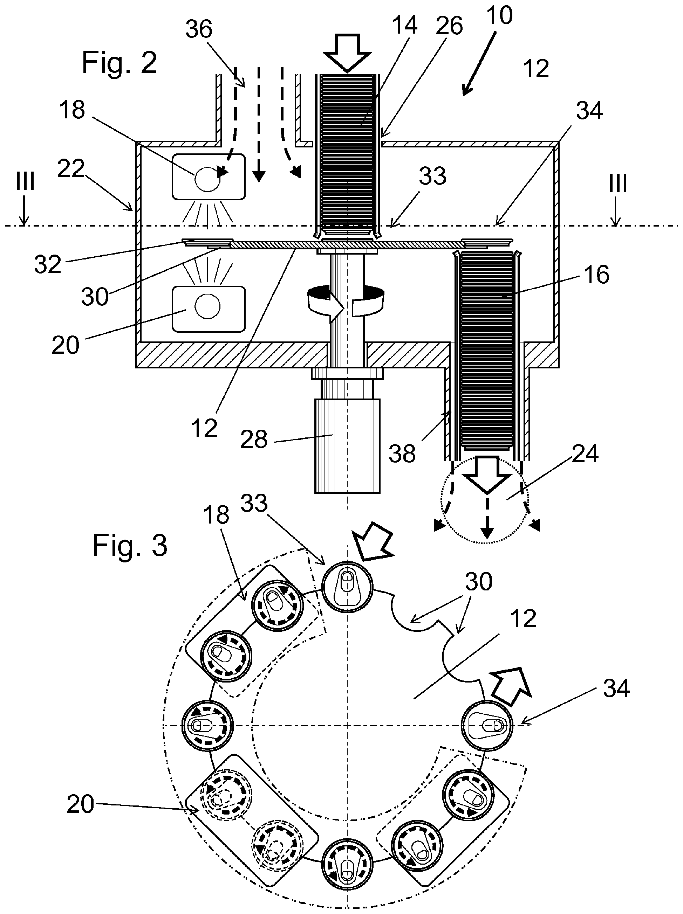

In Fig. 2 ist mit den gestrichelten Pfeilen der Verlauf des keimfreien Gases dargestellt. Dies kann z.B. Sauerstoff oder CO₂ oder irgendein beliebiges sterilisierendes Gas sein.

### Bezugszeichenliste

- 10: Anordnung zum Desinfizieren von Dosendeckeln und Verschließen von Dosen
- 12: Zufuhrvorrichtung - Transportstern
- 14: erste Stapeleinrichtung
- 16: zweite Stapeleinrichtung
- 18: oberer UV-Strahler - erste Sterilisationseinrichtung
- 20: unterer UV-Strahler - zweite Sterilisationseinrichtung
- 22: Gehäuse
- 24: Dosenverschließer
- 26: Zufuhröffnung für Dosendeckel
- 28: Antriebsmotor
- 30: Transportausnehmung im Transportstern für vereinzelte Dosendeckel
- 32: Dosendeckel
- 33: Zufuhrpunkt der Förderstrecke
- 34: Abfuhrpunkt der Förderstrecke
- 36: Gaszufuhrkanal
- 38: Ausgangsrohr

## Patentansprüche

1. Anordnung zum Desinfizieren von Dosendeckeln (32) und zum Verschließen von Dosen umfassend eine Zufuhrvorrichtung (12), in welcher Aufnahmen (30) für vereinzelte Dosendeckel (32) angeordnet sind, welche Zufuhrvorrichtung (12) im Zufuhrbereich eines Dosenverschließers (24) angeordnet ist, wobei wenigstens eine Sterilisationseinrichtung (18, 20) für in der Zufuhrvorrichtung (12) aufgenommene vereinzelte Dosendeckel (32) im Transportbereich der Zufuhrvorrichtung (12) angeordnet ist, wobei in einem Eingang (33) der Zufuhrvorrichtung (12) eine erste Stapeleinrichtung für Dosendeckel (32) angeordnet ist,
**dadurch gekennzeichnet,**
**dass** in einem Ausgang (34) der Zufuhrvorrichtung (12) eine zweite Stapeleinrichtung (16) angeordnet ist, die in der Zufuhr des Dosenverschließers (24) liegt, und dass die Zufuhrvorrichtung (12) einen Transportstern (28) umfasst, und dass die Sterilisationseinrichtung zumindest eine Puls-Licht-Vorrichtung (18, 20) aufweist und dass jeweils eine Puls-Licht -Vorrichtung (18, 20) über und/oder unter der Zufuhrvorrichtung (12) ausgebildet ist, wobei die Puls-Licht-Vorrichtung berührungslos über Lichtimpulse im UV-Bereich die Dosendeckel (32) bestrahlt und damit für eine Keimfreiheit sorgt.

2. Anordnung nach Anspruch 1, **dadurch gekennzeichnet, dass** die Sterilisationseinrichtung jeweils einen Sterilisierer, insbesondere UV-Strahler (18, 20) für die Deckelober- und -unterseite aufweist.

3. Anordnung nach einem der vorhergehenden Ansprüche, **dadurch gekennzeichnet, dass** der Dosenverschließer (24) in dem Transportbereich der Zufuhrvorrichtung (12) angeordnet ist.

4. Anordnung nach einem der vorhergehenden Ansprüche, **dadurch gekennzeichnet, dass** der Transportstern (28) auf seinem Umfangsabschnitt Transportausnehmungen (30) für vereinzelte Dosendeckel (32) aufweist.

5. Anordnung nach Anspruch 1, **dadurch gekennzeichnet, dass** die zweite Stapeleinrichtung (16) den Eingang des Dosenverschließers (24) bildet.

6. Anordnung nach einem der vorhergehenden Ansprüche, **dadurch gekennzeichnet, dass** der Dosenverschließer (24) unmittelbar in oder am Ausgang (34) der Zufuhrvorrichtung (12) angeordnet ist.

7. Anordnung nach einem der vorhergehenden Ansprüche, **dadurch gekennzeichnet, dass** der Dosenverschließer (24) an der Zufuhrvorrichtung (12) angeordnet ist, und die Zufuhrvorrichtung (12) dem Dosenverschließer (24) die sterilisierten Dosendeckel (32) vereinzelt zuführt.

8. Anordnung nach einem der vorhergehenden Ansprüche, **dadurch gekennzeichnet, dass** die Zufuhrvorrichtung (12) und die Sterilisationseinrichtung (18, 20) in einem geschlossenen Gehäuse (22) angeordnet sind, welches eine Gaszufuhr (36) und eine Gasabfuhr (38) für ein keimfreies Gas aufweist, als auch Zu- und Abfuhröffnungen (26, 38) für die Dosendeckel (32).

9. Anordnung nach Anspruch 8, **dadurch gekennzeichnet, dass** die Zufuhröffnung für die Dosendeckel und die Gaszufuhr und/oder die Abfuhröffnung (38) für die Dosendeckel und die Gasabfuhr (38) integriert sind.

10. Anordnung nach Anspruch 8 oder 9, **dadurch gekennzeichnet, dass** die zweite Stapeleinrichtung (16) in der Gasabfuhr (38) angeordnet ist.

## Claims

1. An arrangement for disinfecting can lids (32) and for sealing cans comprising a feed device (12), in which recesses (30) are arranged for individual can lids (32), which feed device (12) is arranged in the feed area of a can sealer (24), wherein at least one sterilisation device (18, 20) for individual can lids (32) received in the feed device (12) is arranged in the transport area of the feed device (12), wherein a first stacker for can lids (32) is arranged in an inlet (33) of the feed device (12),
**characterised in that**
a second stacker (16) is arranged in the outlet (34) of the feed device (12) and lies in the feed of the can sealer (24), and that the feed device (12) comprises a transport star (28), and the sterilisation device comprises at least one pulse-light-device (18, 20) and that in each case a pulse-light-device (18, 20) above and/or below the feed device (12), wherein the pulse-light-device irradiates the can lids (32) contactless via light pulses in the UV range and thus ensures freedom from germs.

2. The arrangement according to claim 1, **characterised in that** the sterilisation device in each case comprises a steriliser, in particular a UV radiator (18, 20) for the can upper and underside.

3. The arrangement according to any one of the preceding claims, **characterised in that** the can sealer (24) is arranged in the transport area of the feed device (12).

4. The arrangement according to any one of the preceding claims, **characterised in that** the transport star (28) has transport recesses (30) for individual can lids (32) on its circumferential section.

5. The arrangement according to claim 1, **characterised in that** the second stacker (16) forms the inlet of the can sealer (24).

6. The arrangement according to any one of the preceding claims, **characterised in** the can sealer (24) is arranged directly in or on the outlet (34) of the feed device (12).

7. The arrangement according to any one of the preceding claims, **characterised in that** the can sealer (24) is arranged on the feed device (12), and that the feed device (12) feeds the sterilised can lids (32) individually to the can sealer (24).

8. The arrangement according to any one of the preceding claims, **characterised in that** the feed device (12) and the sterilization device (18, 20) are arranged in a closed housing (22), which has a gas supply (36) and a gas discharge (38) for a germ-free gas, as well as feed and discharge openings (26, 38) for the can lids (32).

9. The arrangement according to claim 8, **characterised in that** the feed opening for the can lids and the gas supply and/or the discharge opening (38) for the can lids and the gas discharge (38) are integrated.

10. The arrangement according to claim 8 or 9,
**characterised in that** the second stacker (16) is arranged in the gas discharge (38).

## Revendications

1. Ensemble, destiné à désinfecter des couvercles (32) de boîtes et à fermer des boîtes, comprenant un dispositif d'alimentation (12), dans lequel sont placés des réceptacles (30) pour des couvercles (32) de boîtes désolidarisés, lequel dispositif d'alimentation (12) est placé dans la zone d'alimentation d'une fermeuse (24) de boîtes, au moins un système de stérilisation (18, 20) pour des couvercles (32) de boîtes désolidarisés réceptionnés dans le dispositif d'alimentation (12) étant placé dans la zone de transport du dispositif d'alimentation (12), dans une entrée (33) du dispositif d'alimentation (12) étant placé un premier système d'empilage pour des couvercles (32) de boîtes,
**caractérisé**
**en ce que** dans une sortie (34) du dispositif d'alimentation (12) est placé un deuxième système d'empilage (16), qui se situe dans l'alimentation de la fermeuse (24) de boîtes et **en ce que** le dispositif d'alimentation (12) comprend une système de transport (28) en étoile et **en ce que** le système de stérilisation comporte au moins un dispositif de lumière pulsée (18, 20) et en que chaque fois un dispositif de lumière pulsée (18, 20) est conçu au-dessus et / ou en-dessous du dispositif d'alimentation (12), le dispositif de lumière pulsée irradiant sans contact les couvercles (32) de boîtes par l'intermédiaire d'impulsions lumineuse dans le spectre des UV et assurant ainsi une absence de germes.

2. Ensemble selon la revendication 1, **caractérisé en ce que** le système de stérilisation comprend chaque fois un stérilisateur notamment un émetteur d'UV (18, 20) pour les faces supérieures et inférieures des couvercles.

3. Ensemble selon l'une quelconque des revendications précédentes, **caractérisé en ce que** la fermeuse (24) de boîtes est placée dans la zone de transport du dispositif d'alimentation (12).

4. Ensemble selon l'une quelconque des revendications précédentes, **caractérisé en ce que** sur sa partie périphérique, le système de transport (28) en étoile comporte des évidements de transport (30) pour des couvercles (32) de boîtes désolidarisés.

5. Ensemble selon la revendication 1, **caractérisé en ce que** le deuxième système d'empilage (16) constitue l'entrée de la fermeuse (24) de boîtes.

6. Ensemble selon l'une quelconque des revendications précédentes, **caractérisé en ce que** la fermeuse (24) de boîtes est placée directement dans ou sur la sortie (34) du dispositif d'alimentation (12).

7. Ensemble selon l'une quelconque des revendications précédentes, **caractérisé en ce que** la fermeuse (24) de boîtes est placée sur le dispositif d'alimentation (12) et **en ce que** le dispositif d'alimentation (12) alimente un à un vers la fermeuse (24) de boîtes les couvercles (32) de boîtes stérilisés.

8. Ensemble selon l'une quelconque des revendications précédentes, **caractérisé en ce que** le dispositif d'alimentation (12) et le système de stérilisation (18, 20) sont placés dans un carter (22) fermé, lequel comporte une alimentation de gaz (36) et une évacuation de gaz (38) pour un gaz aseptisé, ainsi qu'également des orifices (26, 38) d'alimentation et d'évacuation pour les couvercles (32) de boîtes.

9. Ensemble selon la revendication 8, **caractérisé en ce que** l'orifice d'alimentation pour les couvercles de boîtes et l'alimentation de gaz et / ou l'orifice d'évacuation (38) pour les couvercles de boîtes et l'évacuation de gaz (38) sont intégrés.

10. Ensemble selon la revendication 8 ou 9, **caractérisé en ce que** le deuxième système d'empilage (16) est placé dans l'évacuation de gaz (38).
